(19) Europäisches Patentamt
European Patent Office
Office européen des brevets

(11) Veröffentlichungsnummer: **0 014 963**
**B1**

(12) # EUROPÄISCHE PATENTSCHRIFT

(45) Veröffentlichungstag der Patentschrift: **13.07.83**

(21) Anmeldenummer: **80100747.7**

(22) Anmeldetag: **14.02.80**

(51) Int. Cl.³: **C 07 D 311/74,**
C 07 D 311/94,
C 07 D 309/12,
C 07 D 307/20,
C 07 C 49/517

(54) **Verfahren zur Herstellung von bicyclischen Enoläthern sowie Äther von 2-(3-Hydroxyprop-1-yl)-cycloalkanonen.**

(30) Priorität: **19.02.79 DE 2906296**

(43) Veröffentlichungstag der Anmeldung:
**03.09.80 Patentblatt 80/18**

(45) Bekanntmachung des Hinweises auf die Patenterteilung:
**13.07.83 Patentblatt 83/28**

(84) Benannte Vertragsstaaten:
**BE CH DE FR GB IT NL**

(56) Entgegenhaltungen:
**DE - A - 2 136 496**

**Tetrahedron Letters No. 28, S. 2461—64 (1978)**
**Allinger et al, Org. Chemie, Berlin—New York 1980, S. 992—993 (gutachtlich, nachpubl.)**
**J. F. W. McOmie: Protective Groups in Org. Chem., London—New York (1973) S. 104 u. Blatt: "Abbreviations."**

(73) Patentinhaber: **BASF Aktiengesellschaft**
**Carl-Bosch-Strasse 38**
**D-6700 Ludwigshafen (DE)**

(72) Erfinder: **Kropp, Rudolf**
**Sprottauer Strasse 2**
**D-6703 Limburgerhof (DE)**
Erfinder: **Thoemel, Frank, Dr.**
**Leberstrasse 17**
**D-6940 Weinheim (DE)**
Erfinder: **Nuerrenbach, Axel, Dr.**
**Koenigsberger Strasse 7**
**D-6718 Gruenstadt 1 (DE)**
Erfinder: **Hoffmann, Werner, Dr.**
**Ringstrasse 11 C**
**D-6701 Neuhofen (DE)**
Erfinder: **Wenisch, Franz, Dr.**
**Anselm-Feuerbach-Strasse 14**
**D-6710 Frankenthal (DE)**
Erfinder: **Fuchs, Hartwig**
**Saarlandstrasse 56**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 014 963 B1

Verfahren zur Herstellung von bicyclischen Enoläthern sowie Äther von 2-(3-Hydroxyprop-1-yl)-cycloalkanonen

Die vorliegende Erfindung betrifft ein verbessertes Verfahren zur Herstellung bicyclischer Enoläther der allgemeinen Formel I

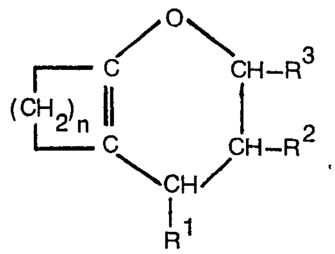

(I)

in der n einen Wert von 3—12 hat und in der die Reste $R^1$—$R^3$ für Wasserstoff oder einen $C_1$—$C_4$-Alkylrest stehen. Ferner betrifft die Erfindung neue Äther von 2-(3-Hydroxyprop-1-yl)-cycloalkanen der allgemeinen Formel II

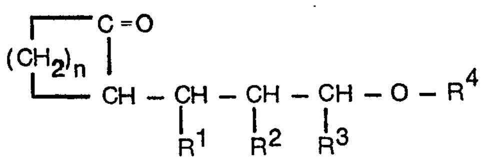

(II)

in der $R^4$ die tert.-Butylgruppe, die Tetrahydrofuran-2-yl-gruppe oder die Tetrahydropyran-2-ylgruppe bedeutet.

Aus der DE—PS 21 36 496 ist es bekannt, die Verbindungen I, die als Zwischenprodukte für die Synthese von moschusartigen Duftstoffen Bedeutung haben, durch radikalische Addition eines Allylalkohols oder eines Allylesters (III')

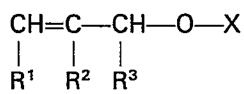

(III')

X = H; —CO-Alkyl

an ein cyclisches Keton (IV)

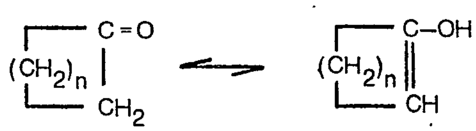

(IV)

und durch sauer katalysierten Ringschluß der hierbei erhaltenen Addukte II' herzustellen

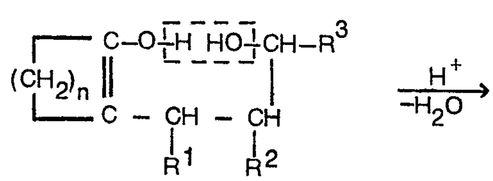


(II') (I)

wobei man im Falle der Verwendung von Allylestern den Säurerest vor der Ringschlußreaktion zuvor hydrolytisch abspalten muß.

Dieses Verfahren ist wegen der großen Empfindlichkeit des Allylalkohols und seiner Homologen nachteilig. Geht man hingegen von den Estern dieser Alkohole aus, so erfordert dies nach der Additionsreaktion den zusätzlichen Verfahrensschritt der hydrolytischen Abspaltung der Säure.

Ferner ist aus Isvest. Akad. Nauk, SSSR, Otdel. Khim. Nauk 1961, Seite 2065 f die radikalische $\alpha$-Addition von Allyläthern von n-Alkan-1-olen an Cyclopentanon und Cyclohexanon zu Verbindungen des Typs (II) bekannt, jedoch lassen sich diese Verbindungen nicht zu bicyclischen Enoläthern cyclisieren.

Der Erfindung lag daher die Aufgabe zugrunde, das aus der DE—PS 21 36 496 in seinen Grundzügen bekannte Verfahren unter Vermeidung der genannten Nachteile zu verbessern.

Es wurde nun gefunden, daß man bicyclische Enoläther der allgemeinen Formel I

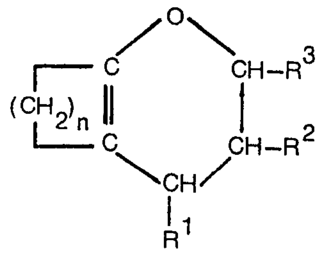

(I)

in der n einen Wert von 3—12 hat und in der die Reste $R^1$—$R^3$ für Wasserstoff oder einen $C_1$—$C_4$-Alkylrest stehen, durch radikalische Addition einer Allylverbindung an ein cyclisches Keton der allgemeinen Formel IV

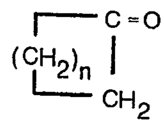

(IV)

und anschließende sauer katalysierte Cyclisierung zu (I) auf vorteilhafte Weise erhält, wenn man als Allylverbindung einen Allyläther der allgemeinen Formel III

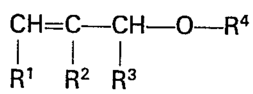

(III)

verwendet, in welcher $R^4$ die tert.-Butylgruppe, die Tetrahydrofuran-2-yl-gruppe oder die Tetrahydropyran-2-yl-gruppe bedeutet.

Die Ausgangsverbindungen (III) leiten sich, entsprechend den hauptsächlich gewünschten Verfahrensprodukten I, vor allem vom Allylalkohol ab. Daneben haben besonders das But-2-en-1-ol, das But-1-en-3-ol und der Methallylalkohol Bedeutung.

Die definitionsgemaßen Äther dieser Alkohole sind in einfacher Weise durch Umsetzung der Alkohole mit Isobuten, 2,3-Dihydrofuran bzw. 2,3-Dihydropyran in Gegenwart saurer Katalysatoren erhältlich, wobei es besonders zweckmäßig ist, als Katalysatoren saure Ionenaustauscher zu verwenden. Man nimmt diese Verätherung im allgemeinen bei Normaldruck oder bei erhöhtem Druck — etwa bis zu 50 bar — und bei Temperaturen von 0—100°C, vorzugsweise 40—80°C, vor.

Unter den Ausgangsverbindungen (IV) sind das Cyclopentanon, Cyclohexanon, Cyclooctanon und vor allem das Cyclododecanon hervorzuheben.

Die radikalischen Katalysatoren für die Umsetzung von (III) mit (IV) können prinzipiell beliebig sein, jedoch verwendet man vorzugsweise solche, die erst bei denjenigen Temperaturen aktiv werden, d.h. in Radikale zerfallen, bei denen die $\alpha$-Addition von (III) an (IV) mit genügender Geschwindigkeit verläuft. Diese Temperaturen liegen in der Regel zwischen 80 und 180°C, besonders zwischen 120 und 150°C. Allgemein läßt sich diese Reaktion bei 50—200°C ausführen, wenngleich sie sich bei tieferen Temperaturen als 80°C merklich verlangsamt und wenngleich man über 150°C in der Regel mit der vermehrten Bildung von Nebenprodukten zu rechnen hat. Für die Wahl der Temperatur ist es weiterhin maßgebend, daß man die Umsetzung nach Möglichkeit bei Normaldruck ausführt, weil sowohl geringerer Druck — etwa bis zu 200 mbar absolut — als auch höherer Druck — etwa bis zu 50 bar — den wirtschaftlichen Nachteil der Verwendung von Druckapparaturen bedingt.

Als Katalysatoren für den besonders bevorzugten Temperaturbereich von 120—150°C eignen sich aus den genannten Gründen beispielsweise Dibenzoylperoxid, tert.-Butylperoctoat und Azodiisobutyronitril sowie besonders Di-tert.-butylperoxid.

(III) und (IV) reagieren in äquimolaren Mengenverhältnissen miteinander, jedoch ist es im allgemeinen zur Unterdrückung von Nebenreaktionen vorteilhaft, (IV) im etwa bis zu 20-fachen molaren Überschuß einzusetzen. Die Menge des radikalischen Katalysators liegt vorzugsweise im Bereich von 0,05—0,7 mol, besonders 0,1—0,3 mol pro Mol (III).

Soweit (III) und (IV) bei den Reaktionstemperaturen flüssig sind und der Katalysator im Reaktionsgemisch ausreichend löslich ist, erübrigt sich in aller Regel die Mitverwendung eines inerten Lösungsmittels. Möchte man trotzdem in Gegenwart eines Lösungsmittels arbeiten, etwa weil die Ausgangsverbindungen bereits in Lösung vorliegen oder weil sich die Bildung von Nebenprodukten mit Hilfe von Lösungsmitteln zuweilen vermindern läßt, so eignen sich hierfür z.B. Petroläther, Cyclohexan, Benzol und Chlorbenzol.

Bei der Umsetzung von (III) mit (IV) erhält man die Verbindungen (II)

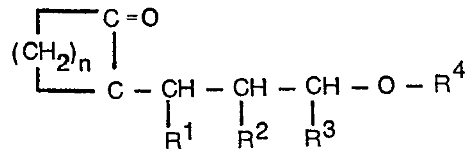

(II)

die man gewünschtenfalls wie üblich isolieren kann, vorzugsweise jedoch unmittelbar der sauer katalysierten Cyclisierungsreaktion unterwirft, welche unter Abspaltung von Wasser sowie von Isobuten, 2,3-Dihydrofuran bzw. 2,3-Dihydropyran verläuft.

Unter den Verbindungen II ist eine der Verbindungen als

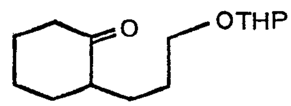

in Tetrahedron Letters No. 28 (1978), Seite 2461—64 beschreiben, wobei als Bedeutung für THP gemäß "Protective Groups in Organic Chemistry", Plenum Press, London und New York, 1973, Seite IX, der 2-Tetrahydropyranylrest anzunehmen ist. Diese Verbindung wurde jedoch lediglich im Zusammenhang mit der Herstellung des 1 - Methylen - 2 - (3 - hydroxyprop - 1 - yl) - cyclohexanons erwähnt, so daß ein Bezug zu der vorliegenden erfindungsgemäßen Lehre nicht gegeben ist.

Als Säuren lassen sich grundsätzlich beliebige Säuren, also Protonensäuren und Lewissäuren, in homogener oder heterogener Phase (z.B. saure Ionenaustauscher) verwenden. Aus verfahrenstechnischen Gründen haben sich jedoch schwerflüchtige organisch starke Säuren in homogener Phase besonders bewährt, wobei die billige p-Toluolsulfonsäure an erster Stelle zu nennen ist.

Die Menge der Säure beträgt zweckmäßigerweise 0,001—0,3, besonders 0,05—0,2 moläqu. pro Mol (II).

Vorzugsweise nimmt man die Cyclisierung bei 60—150°C, besonders bei 80—130°C und unter einem Druck von 0,05 mbar — 50 bar, besonders von 0,1—1 mbar vor. Die Entfernung des Wassers kann durch laufende Azeotropdestillation, z.B. mit Toluol, begünstigt werden. Die olefinischen Spaltprodukte lassen sich nach Abtrennung des Wassers wieder in die Stufe der Herstellung von (III) zurückführen.

Sieht man von der erfindungsgemäßen Verbesserung ab, so läßt sich das Verfahren sowohl diskontinuierlich als auch kontinuierlich nach den üblichen Techniken ausführen, so daß sich nähere Angaben hierzu erübrigen. Dies gilt auch für die Aufarbeitung auf die Verfahrensprodukte (I).

Das Verfahren bietet vor allem den Vorteil, daß man die Allylalkohole (III') bei relativ niedrigen Temperaturen in die Äther (III) überführt, die bei den Reaktionstemperaturen ergänzender Text, einzufügen als Absatz nach Zeile 5 von Seite 6 der radikalischen Addition wesentlich leichter zu handhaben sind als die freien Alkohole. Trotz der zusätzlichen Verätherungsstufe gestaltet sich das Verfahren wirtschaftlicher als bei unmittelbarer Verwendung der Alkohole (III').

## Beispiel 1

Herstellung von 13-Oxabicyclo-[10.4.0]-hexadec-Δ-1,12-en

1820 g (10 mol) Cyclododecanon wurden unter Stickstoffatmosphäre bei 140°C im Laufe von 4 Stunden mit einer Lösung aus 114 g (1 mol) Allyl-tert.-butyläther und 29,2 g (0,2 mol) Di-tert.-butylperoxid versetzt und noch eine weitere Stunde bei dieser Temperatur gehalten. Das überschüssige Cyclododecanon wurde sodann abdestilliert, wonach der Rückstand zusammen mit 10 g (58 mmol) p-Toluolsulfonsäure eine Stunde lang bei 300 mbar auf 130°C erhitzt wurde. Hierbei wurden 0,7 mol Wasser und 0,7 mol Isobuten abgespalten. Die übliche Aufarbeitung lieferte die oben genannte Verbindung in 56%iger Ausbeute.

Sdp. 112—114°C/0,1 mbar; $n_D^{20} = 1.5079$

## Beispiel 2

Herstellung von 13-Oxabicyclo-[10.4.0]-hexadec-Δ-1,12-en

Cyclododecanon und Allyl-tert.-butyläther wurden analog Beispiel 1 im Laufe von 6 Stunden miteinander umgesetzt. Nach Entfernung des überschüssigen Cyclododecanons wurde der Rückstand zusammen mit 10 g p-Toluolsulfonsäure und 1 l Toluol bei Normaldruck 3 Stunden lang unter Rückflußkühlung erhitzt, wobei das Wasser laufend azeotrop abdestilliert wurde. Die Lösung wurde mit verdünnter wäßriger $NaHCO_3$-Lösung neutral gewaschen und danach wie üblich aufgearbeitet. Die Ausbeute an dem oben genannten Verfahrensprodukt betrug 65%.

Beispiel 3

2-(3-tert.-Butoxy-prop-1-yl)-cyclopentanon

638 g (7,6 mol) Cyclopentanon wurden unter Stickstoffatmosphäre bei 125—130°C im Laufe von 4 Stunden mit einer Lösung aus 87 g (0,76 mol) Allyl-tert.-butyläther und 33 g (0,23 mol) Di-tert.-butylperoxid versetzt und anschließend noch 4 Stunden auf 120°C gehalten.

Die destillative Aufarbeitung des Reaktionsgemisches lieferte das 2-(3-tert.-Butoxy-prop-1-yl)-cyclopentanon in 50%iger Ausbeute.

Sdp. 95—90°C/0,4 mbar; $n_D^{24,5} = 1,4517$

Beispiel 4

Herstellung von 6-Oxabicyclo-[3,4,0]-non-Δ-1,5-en

37 g (0,19 mol) des Verfahrensproduktes von Beispiel 3 wurden zusammen mit 5 g (29 mmol)p-Toluolsulfonsäure 1 Stunde lang bei 18 mbar auf 100°C erhitzt. Danach wurde das Reaktionsgemisch wie üblich aufgearbeitet; die Ausbeute an dem oben genannten bicyclischen Enoläther betrug 30%, bezogen auf den eingesetzten Allyläther.

Sdp. 68—70°C/0,15 mbar

Beispiel 5

2-(3-tert.-Butoxy-prop-1-yl)-cyclohexanon

980 g (10 mol) Cyclohexanon wurden unter Rühren bei 140°C im Laufe von 2 Stunden mit einer Lösung aus 114 g (1 mol) Allyl-tert.-butyläther und 29,2 g (0,2 mol) Di-tert.-butylperoxid versetzt und noch eine Stunde bei dieser Temperatur gehalten. Die destillative Aufarbeitung des Reaktionsgemisches lieferte das 2-(3-tert.-Butoxy-prop-1-yl)-cyclohexanon in 60%iger Ausbeute.

Sdp. 85—90°C/0,05 mbar; $n_D^{24,5} = 1,4553$

Beispiel 6

Herstellung von 7-Oxabicyclo-[4.4.0]-dec-Δ-1,5-en

180 g (0,85 mol) des Verfahrensproduktes von Beispiel 5 wurden zusammen mit 10 g (58 mmol) p-Toluolsulfonsäure und 1 l Toluol analog Beispiel 2 der Cyclisierungsreaktion unterworfen. Die Ausbeute an dem oben genannten Verfahrensprodukt betrug 42%, bezogen auf den Allyläther.

Sdp. 66—70°C/0,05 mbar; $n_D^{24,5} = 1,4932$

Beispiel 7

2-(3-tert.-Butoxy-prop-1-yl)-cyclooctanon

504 g (4 mol) Cyclooctanon, 46 g (0,4 mol) Allyl-tert.-butyläther und 23 g (0,16 mol) Di-tert.-butylperoxid wurden analog Beispiel 1 umgesetzt. Die Ausbeute an 2-(3-tert.-Butoxy-prop-1-yl)-cyclooctanon betrug 84%.

Sdp. 105—110°C/0,15 mbar; $n_D^{24,5} = 1,4681$

Beispiel 8

Herstellung von 9-Oxabicyclo-[6.4.0]-dodec-Δ-1,8-en

49 g (0,2 mol) des Verfahrensprodukts von Beispiel 7 wurden zusammen mit 5 g (29 mmol) p-Toluolsulfonsäure und 0.5 l Toluol auf die in Beispiel 2 beschriebene Weise der Cyclisierungsreaktion unterworfen. Die Aufarbeitung lieferte die oben genannte Verbindung in 75%iger Ausbeute, bezogen auf den Allyläther.

Sdp-. 55°C/0,15 mbar; $n_D^{24,5} = 1,4938$

Beispiel 9

Herstellung von 13-Oxabicyclo-[10.4.0]-hexadec-Δ-1,12-en

237 g (1,3 mol) Cyclododecanon wurden bei 140°C im Laufe von 6 Stunden mit 142 g (1 mol) 2-Allyloxytetrahydropyran und 56 g (0.38 mol) Di-tert.-butylperoxid versetzt und anschließend noch 5 Stunden bei dieser Temperatur gehalten.

Nach Entfernung des überschüssigen Cyclododecanons wurde das Additionsprodukt zusammen mit 1 g (6 mmol) p-Toluolsulfonsäure bei 0,1 mbar auf 125°C erhitzt, wobei die oben genannte Verbindung überdestillierte und in 65%iger Ausbeute anfiel.

Unter gleichen Bedingungen, jedoch mit 10 mol Cyclodecanon konnte die Ausbeute auf 75% erhöht werden.

Beispiel 10

Herstellung von 7-Oxabicyclo-[4.4.0]-dec-Δ-1,6-en

980 g (10 mol) Cyclohexanon, 142 g (1 mol) 2-(Allyloxy)-tetrahydropyran und 56 g (0,38 mol) Di-tert.-butylperoxid wurden analog Beispiel 6 zu der oben genannten Verbindung umgesetzt; die Ausbeute betrug 55%.

Beispiel 11

Herstellung von 14-Methyl-13-oxabicyclo-[10.4.0]-hexadec-Δ-1,12-en

910 g (5 mol) Cyclododecanon, 78 g (0,5 mol)-2-(But-1-en-3-yloxy)-tetrahydropyran und 28 g (0,18 mol) Di-Tert.-butylperoxid wurden analog Beispiel 6 zu der oben genannten Verbindung umgesetzt. Die Ausbeute betrug 44%.

Sdp. 110—112°C/0,1 mbar.

Beispiel 12

Herstellung von 13-Oxabicyclo-[10.4.0]-hexadec-Δ-1,12-en

455 g (2,5 mol) Cyclododecanon wurden unter Stickstoffatmosphäre bei 100°C im Laufe von 4 Stunden mit einer Lösung aus 57 g (0.5 mol) Allyl-tert.-butyläther und 23 g (0,1 mol) tert.-Butylperoctoat versetzt und noch eine weitere Stunde bei dieser Temperatur gehalten. Das überschüssige Cyclododecanon wurde sodann abdestilliert, wonach der Rückstand zusammen mit 3 g (17,4 mmol) p-Toluolsulfonsäure eine Stunde lang bei 20 mbar auf 100°C erhitzt wurde. Danach wurde das Reaktionsgemisch wie üblich aufgearbeitet; die Ausbeute an dem oben genannten bicyclischen Enoläther betrug 30% bezogen auf den Allyläther.

Beispiel 13

Kontinuierliche Herstellung von 13-Oxabicyclo-[10.4.0]-hexadec-Δ-1,12-en

Eine Lösung aus 1 400 g Cyclohexan, 310 g (5 mol) Cyclododecanon, 114 g (1 mol) Allyl-tert.-butyläther und 58,4 g (0,4 mol) Di-tert.-butylperoxid wurden im Verlauf von 25 Stunden bei 150°C und 18 bar durch einen 0,3-1-Rührautoklaven gepumpt. Aus dem Reaktionsaustrag wurde das Lösungsmittel und das überschüssige Cyclododecanon abdestilliert, wonach der Rückstand zusammen mit 15 g (87 mmol) p-Toluolsulfonsäure eine Stunde lang bei 300 mbar auf 120°C erhitzt wurde. Die übliche Aufarbeitung lieferte die oben genannte Verbindung in 40%iger Ausbeute.

**Patentansprüche**

1. Verfahren zur Herstellung von bicyclischen Enoläthern der allgemeinen Formel I

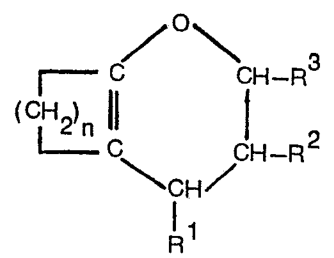

(I)

in der n einen Wert von 3—12 hat und in der die Reste $R^1$—$R^3$ für Wasserstoff oder einen $C_1$—$C_4$-Alkylrest stehen, durch radikalische Addition einer Allylverbindung an ein cyclisches Keton der allgemeinen Formel IV

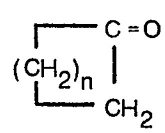

(IV)

und anschließende sauer katalysierte Cyclisierung zu (I), dadurch gekennzeichnet, daß man als Allylverbindung einen Allyläther der allgemeinen Formel III

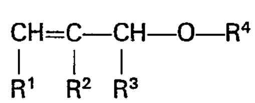

(III)

verwendet, in welcher $R^4$ die tert.-Butylgruppe, die Tetrahydrofuran-2-yl-gruppe oder die Tetrahydropyran-2-yl-gruppe bedeutet.

2. Äther von 2-(3-Hydroxy-prop-1-yl)-cycloalkanonen der allgemeinen Formel II

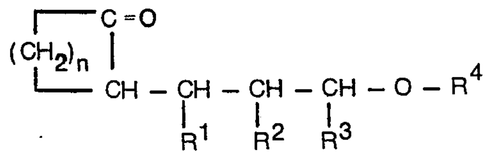

(II)

in der $R^4$ die tert.-Butylgruppe, die Tetrahydrofuran-2-yl-gruppe oder die Tetrahydropyran-2-yl-gruppe bedeutet, ausgenommen das 2-[3-(2-Tetrahydropyranyl)-oxyprop-1-yl]-cyclohexanon.

**Revendications**

1. Procédé de préparation d'éthers d'énols bicycliques de formule générale I

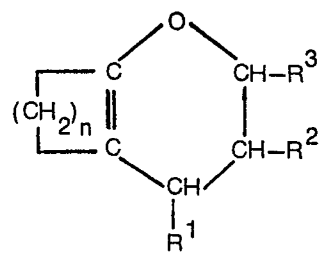

(I)

dans laquelle n a une valeur de 3 à 12 et dans laquelle les symboles $R^1$ à $R^3$ représentent l'hydrogène ou un groupe alkyle en C1—C4, par addition radicalaire d'un composé allylique sur une cétone cyclique de formule générale IV

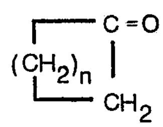

(IV)

suivie d'une cyclisation catalysée par un acide donnant le composé I, caractérisé en ce que l'on utilise en tant que composé allylique un éther allylique de formule générale III

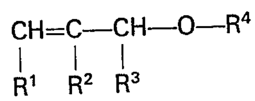

(III)

dans laquelle $R^4$ représente le groupe tert.-butyle, le groupe tétrahydrofuranne-2-yle ou le groupe tétrahydropyranne-2-yle.

2. Les éthers de 2-(3-hydroxy-propyl-1)-cycloalcanones de formule générale II

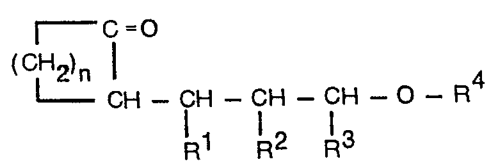

(II)

dans laquelle $R^4$ représente le groupe tert.-butyle, le groupe tétrahydrofuranne-2-yle ou le groupe tétrahydropyranne-2-yle, à l'exception de la 2-[3-(2-tétrahydropyranyl)-oxypropyl-1]-cyclohexanone.

**Claims**

1. A process for the preparation of a bicyclic enol-ether of the general formula I

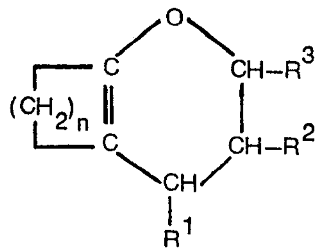

(I)

where n is from 3 to 12 and $R^1$, $R^2$ and $R^3$ are hydrogen or $C_1$—$C_4$-alkyl, by free radical adduct formation of an allyl compound with a cyclic ketone of the general formula IV

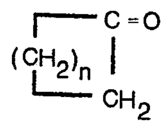

(IV)

and subsequent acid-catalyzed cyclization to give (I), wherein the allyl compound used is an allyl ether of the general formula III

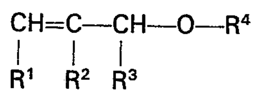

(III)

where $R^4$ is tert.-butyl, tetrahydrofuran-2-yl or tetrahydropyran-2-yl.

2. An ether of a 2-(3-hydroxy-prop-1-yl)-cycloalkanone of the general formula II

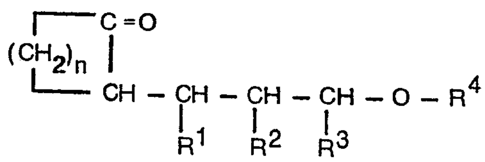

(II)

where $R^4$ is tert.-butyl, tetrahydrofuran-2-yl or tetrahydropyran-2-yl, excluding 2-[3-(tetrahydropyran-2-yl)-oxyprop-1-yl]-cyclohexanone.